# EUROPEAN PATENT APPLICATION

(11) **EP 3 954 777 A1**
(43) Date of publication of application: **16.02.2022**
(21) Application number: 20190525.4
(22) Date of filing: 11.08.2020
(51) Int. Cl.: C12P 17/06, A23L 33/10, C12P 21/00, C12P 7/42, C12N 9/18

(54) **METHOD FOR ENZYMATIC DEGRADATION OF BITTER COMPOUNDS IN AGRO-INDUSTRIAL BY-PRODUCTS**

(71) Applicant: SternEnzym GmbH & Co. KG, 22926 Ahrensburg (DE)
(72) Inventor: BERGER, Ralf G., 30455 Hannover (DE); POPPER, Lutz, 22527 Hamburg (DE)
(74) Representative: Moré, Solveig Helga

(57) **Abstract**

The present invention relates to the use of enzymes in the production of food. In particular, the invention provides novel methods and applications for enzymes for the exploitation of agro-industrial by-products to obtain more palatable animal feed and food products suitable for human consumption. More specifically, the invention discloses a method for enzymatically degrading kaempferol-3-*O*-*(2"'*-*O*-sinapoyl-β-sophoroside), a recently identified bitter-constituent present in rapeseed that has, so far, prevented this plant from serving as a protein source for human nutrition. The invention further teaches the use of enzymes derived from the basidiomycete *Schizophyllum commune* that are capable of hydrolyzing sinapyl ester-containing kaempferol-3-*O*-(2"'-*O*-sinapoyl-β-sophoroside) into non-bitter degradation products. Finally, the invention provides a feed, e.g., an animal feed or a food product for human consumption obtainable by the methods disclosed herein.

SEQUENCE LISTING

<110> SternEnzym GmbH & Co. KG

<120>
Method for enzymatic degradation of bitter compounds in agro-industrial
by-products

<130> 12632 P 6546 EP

<160> 13

<170> BiSSAP 1.3.6

<210> 1
<211> 1500
<212> DNA
<213> Schizophyllum commune

<220>
<223> ScFae1 without endogenous signal sequence (na)

<400> 1
caaggcgact tctccgccaa ctgctcctcc ttcgtcgatg cgatcaccct cgagaacgtc 60

accgtgcagt cgacggagtt cgtcgcggct ggcacgaacg tcagcgtgta cgttcccgag 120

tcgtgcatgt cggcctcgta ccaggtcgtc agcgctgacc tttgccgcgc cacgatgaac 180

gtctcgactt cggaccgcag cggtatccgc ctcgaggcgt ggttcccgca gaactatacc 240

ggtcggttct tgagcacggg caacggcgga attggaggat gcatccagta cagcgacctc 300

gactacgccg cctccctcgg cttcgccgcc gtcggcgcca acaacggcca cgacggcatg 360

accggcgagc ccttcctcaa caaccccgac gtcatcaccg acttcgcctg gcgctccctc 420

cataccggcg tcgtcgtcgg caagcagctc gtcgagacct tctacggcgc cccgcacagc 480

aagtcgtact acctcggctg ctcgaccggc gggcggcagg ggtggaagat ggtgcaggac 540

ttcccggagg actttgatgg cgtagtggcg ggcgcgccgg cgatggcgtt caatgcgctg 600

ttgtattgga gcgggtcgtt ctatacggtc actgggacct cggacgaccc aaccttcgtg 660

ccggtcgact tttgggcgaa caatatccac caggcggtcc ttgatcagtg cgacgcgctc 720

gacggggcga ccgacggtgt attggagaat ccggatctct gcgacttcga cgtgacgcct 780

ctcaactgcg cggacggcaa cacgaccgac tgcttgaccg agacgcaggc ggagaccgtg 840

cgcacggtct tgagcgctat ctatgacgcg aacggcacgc tcgtgtaccc gcgcctgcag 900

ccgggcgcgg aggtcttggc ggggcagcag cagttcaacg gccagccttt cgacatcact 960

caggattggt accgctacgt catctacaac gactcgacct gggatcctgc gacgctgaac 1020

ctcggcgact acgcagtcgc cctcgcgcag aatcccgcca acatcgagac atttaacggc 1080

gacatctccg ctttcgaggc cgcaggaggc aaggtcctgc actaccacgg cctcatggac 1140

ggtctcatct cgtccgacaa ctccaagcgg tactacaagc ttgtgcagga gacgatgggc 1200

aaggactcgt ccgagctcga cgacttctac cgcttcttcc cgatcagcgg catgtcgcac 1260

tgcagcgggg gcgacggggc gtataggatt gggaatgttg atggcggagc ggggacgagt 1320

gcggatgaca atgtgctgat gtcgatggtg cggtgggtgg aggagggcgt ggcgccggaa 1380

gtcgtgcgcg gggcgaatgc taacgtgacc tactggcgtt cgcattgcaa gtggcccctg 1440

acgaacaagt acgttggacc tggatcgtat gaggatgagt ctgcatggca gtgcagctga 1500

<210> 2
<211> 72
<212> DNA
<213> Schizophyllum commune

<220>
<223> Endogenous signal sequence ScFae1 (na)

<400> 2
atggctccta cagtgcgctc tctcctcagc gcgctcggca gcgtgctcgc actcgcgcgc 60

ttcacgaatg cc 72

<210> 3
<211> 1572
<212> DNA
<213> Schizophyllum commune

<220>
<223> ScFae1 with endogenous signal sequence (na)

<400> 3
atggctccta cagtgcgctc tctcctcagc gcgctcggca gcgtgctcgc actcgcgcgc 60

ttcacgaatg cccaaggcga cttctccgcc aactgctcct ccttcgtcga tgcgatcacc 120

ctcgagaacg tcaccgtgca gtcgacggag ttcgtcgcgg ctggcacgaa cgtcagcgtg 180

tacgttcccg agtcgtgcat gtcggcctcg taccaggtcg tcagcgctga cctttgccgc 240

gccacgatga acgtctcgac ttcggaccgc agcggtatcc gcctcgaggc gtggttcccg 300

cagaactata ccggtcggtt cttgagcacg ggcaacggcg gaattggagg atgcatccag 360

tacagcgacc tcgactacgc cgcctccctc ggcttcgccg ccgtcggcgc caacaacggc 420

cacgacggca tgaccggcga gcccttcctc aacaaccccg acgtcatcac cgacttcgcc 480

tggcgctccc tccataccgg cgtcgtcgtc ggcaagcagc tcgtcgagac cttctacggc 540

gccccgcaca gcaagtcgta ctacctcggc tgctcgaccg gcgggcggca ggggtggaag 600

atggtgcagg acttcccgga ggactttgat ggcgtagtgg cgggcgcgcc ggcgatggcg 660

ttcaatgcgc tgttgtattg gagcgggtcg ttctatacgg tcactgggac ctcggacgac 720

ccaaccttcg tgccggtcga cttttgggcg aacaatatcc accaggcggt ccttgatcag 780

tgcgacgcgc tcgacggggc gaccgacggt gtattggaga atccggatct ctgcgacttc 840

gacgtgacgc ctctcaactg cgcggacggc aacacgaccg actgcttgac cgagacgcag 900

gcggagaccg tgcgcacggt cttgagcgct atctatgacg cgaacggcac gctcgtgtac 960

ccgcgcctgc agccgggcgc ggaggtcttg gcggggcagc agcagttcaa cggccagcct 1020

ttcgacatca ctcaggattg gtaccgctac gtcatctaca acgactcgac ctgggatcct 1080

gcgacgctga acctcggcga ctacgcagtc gccctcgcgc agaatcccgc caacatcgag 1140

acatttaacg gcgacatctc cgctttcgag gccgcaggag gcaaggtcct gcactaccac 1200

ggcctcatgg acggtctcat ctcgtccgac aactccaagc ggtactacaa gcttgtgcag 1260

gagacgatgg gcaaggactc gtccgagctc gacgacttct accgcttctt cccgatcagc 1320

ggcatgtcgc actgcagcgg gggcgacggg gcgtatagga ttgggaatgt tgatggcgga 1380

gcggggacga gtgcggatga caatgtgctg atgtcgatgg tgcggtgggt ggaggagggc 1440

gtggcgccgg aagtcgtgcg cggggcgaat gctaacgtga cctactggcg ttcgcattgc 1500

aagtggcccc tgacgaacaa gtacgttgga cctggatcgt atgaggatga gtctgcatgg 1560

cagtgcagct ga 1572

<210> 4
<211> 499
<212> PRT
<213> Schizophyllum commune

<220>
<223> ScFae1 without endogenous signal sequence (aa)

<400> 4
Gln Gly Asp Phe Ser Ala Asn Cys Ser Ser Phe Val Asp Ala Ile Thr
1               5                   10                  15
Leu Glu Asn Val Thr Val Gln Ser Thr Glu Phe Val Ala Ala Gly Thr
            20                  25                  30
Asn Val Ser Val Tyr Val Pro Glu Ser Cys Met Ser Ala Ser Tyr Gln
        35                  40                  45
Val Val Ser Ala Asp Leu Cys Arg Ala Thr Met Asn Val Ser Thr Ser
    50                  55                  60
Asp Arg Ser Gly Ile Arg Leu Glu Ala Trp Phe Pro Gln Asn Tyr Thr
65                  70                  75                  80
Gly Arg Phe Leu Ser Thr Gly Asn Gly Gly Ile Gly Gly Cys Ile Gln
                85                  90                  95
Tyr Ser Asp Leu Asp Tyr Ala Ala Ser Leu Gly Phe Ala Ala Val Gly
            100                 105                 110
Ala Asn Asn Gly His Asp Gly Met Thr Gly Glu Pro Phe Leu Asn Asn
        115                 120                 125
Pro Asp Val Ile Thr Asp Phe Ala Trp Arg Ser Leu His Thr Gly Val
    130                 135                 140
Val Val Gly Lys Gln Leu Val Glu Thr Phe Tyr Gly Ala Pro His Ser
145                 150                 155                 160
Lys Ser Tyr Tyr Leu Gly Cys Ser Thr Gly Gly Arg Gln Gly Trp Lys
                165                 170                 175
Met Val Gln Asp Phe Pro Glu Asp Phe Asp Gly Val Val Ala Gly Ala
            180                 185                 190

Pro Ala Met Ala Phe Asn Ala Leu Leu Tyr Trp Ser Gly Ser Phe Tyr
            195                 200                 205
Thr Val Thr Gly Thr Ser Asp Asp Pro Thr Phe Val Pro Val Asp Phe
    210                 215                 220
Trp Ala Asn Asn Ile His Gln Ala Val Leu Asp Gln Cys Asp Ala Leu
225                 230                 235                 240
Asp Gly Ala Thr Asp Gly Val Leu Glu Asn Pro Asp Leu Cys Asp Phe
                245                 250                 255
Asp Val Thr Pro Leu Asn Cys Ala Asp Gly Asn Thr Thr Asp Cys Leu
            260                 265                 270
Thr Glu Thr Gln Ala Glu Thr Val Arg Thr Val Leu Ser Ala Ile Tyr
        275                 280                 285
Asp Ala Asn Gly Thr Leu Val Tyr Pro Arg Leu Gln Pro Gly Ala Glu
    290                 295                 300
Val Leu Ala Gly Gln Gln Gln Phe Asn Gly Gln Pro Phe Asp Ile Thr
305                 310                 315                 320
Gln Asp Trp Tyr Arg Tyr Val Ile Tyr Asn Asp Ser Thr Trp Asp Pro
                325                 330                 335
Ala Thr Leu Asn Leu Gly Asp Tyr Ala Val Ala Leu Ala Gln Asn Pro
            340                 345                 350
Ala Asn Ile Glu Thr Phe Asn Gly Asp Ile Ser Ala Phe Glu Ala Ala
        355                 360                 365
Gly Gly Lys Val Leu His Tyr His Gly Leu Met Asp Gly Leu Ile Ser
    370                 375                 380
Ser Asp Asn Ser Lys Arg Tyr Tyr Lys Leu Val Gln Glu Thr Met Gly
385                 390                 395                 400
Lys Asp Ser Ser Glu Leu Asp Asp Phe Tyr Arg Phe Phe Pro Ile Ser
                405                 410                 415
Gly Met Ser His Cys Ser Gly Gly Asp Gly Ala Tyr Arg Ile Gly Asn
            420                 425                 430
Val Asp Gly Gly Ala Gly Thr Ser Ala Asp Asp Asn Val Leu Met Ser
        435                 440                 445
Met Val Arg Trp Val Glu Glu Gly Val Ala Pro Glu Val Val Arg Gly
    450                 455                 460
Ala Asn Ala Asn Val Thr Tyr Trp Arg Ser His Cys Lys Trp Pro Leu
465                 470                 475                 480
Thr Asn Lys Tyr Val Gly Pro Gly Ser Tyr Glu Asp Glu Ser Ala Trp
                485                 490                 495
Gln Cys Ser


<210> 5
<211> 24
<212> PRT
<213> Schizophyllum commune


<220>
<223> Endogenous signal sequence ScFae1 (aa)

<400> 5
Met Ala Pro Thr Val Arg Ser Leu Leu Ser Ala Leu Gly Ser Val Leu
1               5                   10                  15
Ala Leu Ala Arg Phe Thr Asn Ala
            20

<210> 6
<211> 523
<212> PRT
<213> Schizophyllum commune


<220>

<223> ScFae1 with endogenous signal sequence (aa)

<400> 6
Met Ala Pro Thr Val Arg Ser Leu Leu Ser Ala Leu Gly Ser Val Leu
1               5                   10                  15
Ala Leu Ala Arg Phe Thr Asn Ala Gln Gly Asp Phe Ser Ala Asn Cys
            20                  25                  30
Ser Ser Phe Val Asp Ala Ile Thr Leu Glu Asn Val Thr Val Gln Ser
        35                  40                  45
Thr Glu Phe Val Ala Ala Gly Thr Asn Val Ser Val Tyr Val Pro Glu
    50                  55                  60
Ser Cys Met Ser Ala Ser Tyr Gln Val Val Ser Ala Asp Leu Cys Arg
65                  70                  75                  80
Ala Thr Met Asn Val Ser Thr Ser Asp Arg Ser Gly Ile Arg Leu Glu
                85                  90                  95
Ala Trp Phe Pro Gln Asn Tyr Thr Gly Arg Phe Leu Ser Thr Gly Asn
            100                 105                 110
Gly Gly Ile Gly Gly Cys Ile Gln Tyr Ser Asp Leu Asp Tyr Ala Ala
        115                 120                 125
Ser Leu Gly Phe Ala Ala Val Gly Ala Asn Asn Gly His Asp Gly Met
    130                 135                 140
Thr Gly Glu Pro Phe Leu Asn Asn Pro Asp Val Ile Thr Asp Phe Ala
145                 150                 155                 160
Trp Arg Ser Leu His Thr Gly Val Val Val Gly Lys Gln Leu Val Glu
                165                 170                 175
Thr Phe Tyr Gly Ala Pro His Ser Lys Ser Tyr Tyr Leu Gly Cys Ser
            180                 185                 190
Thr Gly Gly Arg Gln Gly Trp Lys Met Val Gln Asp Phe Pro Glu Asp
        195                 200                 205
Phe Asp Gly Val Val Ala Gly Ala Pro Ala Met Ala Phe Asn Ala Leu
    210                 215                 220
Leu Tyr Trp Ser Gly Ser Phe Tyr Thr Val Thr Gly Thr Ser Asp Asp
225                 230                 235                 240
Pro Thr Phe Val Pro Val Asp Phe Trp Ala Asn Asn Ile His Gln Ala
                245                 250                 255
Val Leu Asp Gln Cys Asp Ala Leu Asp Gly Ala Thr Asp Gly Val Leu
            260                 265                 270
Glu Asn Pro Asp Leu Cys Asp Phe Asp Val Thr Pro Leu Asn Cys Ala
        275                 280                 285
Asp Gly Asn Thr Thr Asp Cys Leu Thr Glu Thr Gln Ala Glu Thr Val
    290                 295                 300
Arg Thr Val Leu Ser Ala Ile Tyr Asp Ala Asn Gly Thr Leu Val Tyr
305                 310                 315                 320
Pro Arg Leu Gln Pro Gly Ala Glu Val Leu Ala Gly Gln Gln Gln Phe
                325                 330                 335
Asn Gly Gln Pro Phe Asp Ile Thr Gln Asp Trp Tyr Arg Tyr Val Ile
            340                 345                 350
Tyr Asn Asp Ser Thr Trp Asp Pro Ala Thr Leu Asn Leu Gly Asp Tyr
        355                 360                 365
Ala Val Ala Leu Ala Gln Asn Pro Ala Asn Ile Glu Thr Phe Asn Gly
    370                 375                 380
Asp Ile Ser Ala Phe Glu Ala Ala Gly Gly Lys Val Leu His Tyr His
385                 390                 395                 400
Gly Leu Met Asp Gly Leu Ile Ser Ser Asp Asn Ser Lys Arg Tyr Tyr
                405                 410                 415
Lys Leu Val Gln Glu Thr Met Gly Lys Asp Ser Ser Glu Leu Asp Asp
            420                 425                 430
Phe Tyr Arg Phe Phe Pro Ile Ser Gly Met Ser His Cys Ser Gly Gly
        435                 440                 445
Asp Gly Ala Tyr Arg Ile Gly Asn Val Asp Gly Gly Ala Gly Thr Ser
    450                 455                 460
Ala Asp Asp Asn Val Leu Met Ser Met Val Arg Trp Val Glu Glu Gly
465                 470                 475                 480

Val Ala Pro Glu Val Val Arg Gly Ala Asn Ala Asn Val Thr Tyr Trp
                485                 490                 495
Arg Ser His Cys Lys Trp Pro Leu Thr Asn Lys Tyr Val Gly Pro Gly
            500                 505                 510
Ser Tyr Glu Asp Glu Ser Ala Trp Gln Cys Ser
        515                 520

<210> 7
<211> 1500
<212> DNA
<213> Schizophyllum commune

<220>
<223> ScFae3 without endogenous signal sequence (na)

<400> 7
caagacgact tcgcctcaga ctgctcggcg tttatcgaca agataatcct cgacaatgtc 60

actgtcacct cgacggagtt tgtggcagcg ggtacgaact tgactgtgta cgtccccaac 120

tcgtgcgggt cgccgtcgta tcaggtcgtc agcaccgacc tctgccgcgc gactatgaac 180

gtcacgacct ctgagcgcag cggcatacgc ctcgaggcct ggttcccgca gaactacacc 240

ggccgcttct tgagcactgg taacggtggt atcggaggat gcattcagta tagcgacatc 300

gactatgccg cctccctcgg tttcgctgcc gtcggcgcca acaacggcca cgacggcatg 360

accggcgagc ccttcctcaa caacccggac gttgtcaccg acttcgcctg gcgctcgctg 420

cacaccggcg tcgtcgtcgg caagcaactc accgagacct tctacggcgc gccccacaac 480

aagtcgtact acctcggctg ctctactggc ggtcggcagg gcttcaaggc ggtgcaggac 540

tttccggacg acttcgacgg cgtcgtcgcg ggcgcgccgg ccttcgcttt caatgggctg 600

ctgtactgga gcggctcgtt cctgcccgtc accggaacct cggaggaccc gaccttcgtc 660

cccacggatt tctgggcgca gaatatccac gcggaggtcc tccagcaatg tgacgcgctc 720

gacggcgcga cggatggggt cctggagaac ccggacctct gcgacttcga cgtgacgccg 780

ctcatctgca cggacggcaa cacgacgggc tgcctgaccg agacgcaggc ggagaccgtg 840

cgcacggtgc tcagccccat ctacgacaag aacggcaccc tcgtgtaccc gcgtctgcag 900

cctggtgcag aggtcctcgc cgggtttgcc cagttcggcg gccagccctt cctttattct 960

acggactggt accgctacgt cgtctacaac gactcgaatt gggaccccat gacgctctcc 1020

attgacgact acgccgccgc ccgcgcccag aaccccgcca atgtcgagac gttcgagggc 1080

gacatctccg ccttcgcgtc ctccggtggc aaggtcctgc actaccacgg cctgatggac 1140

ggcctgatct cctccgacaa ctccaagcgg tactacgccc tcgttcagca aaccctcaac 1200

caagagcccg ccgagctcga cgagttctac cgcttcttcc ccatcagcgg gatgtcgcac 1260

tgcagcggcg gcgacggcgc gtaccgcatc gggaacgtcg agggcggcgc gggcacgagt 1320

gcggacgaca acgtgctgat gtccatggtg cggtgggtgg aggagggcgt ggcgccggag 1380

gttgtgcgcg gggcggacgc gaatgcgact tattggcgcg cgcattgcaa gtggccgaag 1440

acgaacaagt acgtcggacc gggggattat gaggatgaga gtgcatggga gtgttcttga 1500

<210> 8
<211> 69
<212> DNA
<213> Schizophyllum commune

<220>
<223> Endogenous signal sequence ScFae3 (na)

<400> 8
atggcgcccc tccgctcagt ttctagcgtg ctggtgggtg cgctcgcgct cctgcgcgcc 60

gcgcacgcg 69

<210> 9
<211> 1569
<212> DNA
<213> Schizophyllum commune

<220>
<223> ScFae3 with endogenous signal sequence (na)

<400> 9
atggcgcccc tccgctcagt ttctagcgtg ctggtgggtg cgctcgcgct cctgcgcgcc 60

gcgcacgcgc aagacgactt cgcctcagac tgctcggcgt ttatcgacaa gataatcctc 120

gacaatgtca ctgtcacctc gacggagttt gtggcagcgg gtacgaactt gactgtgtac 180

gtccccaact cgtgcgggtc gccgtcgtat caggtcgtca gcaccgacct ctgccgcgcg 240

actatgaacg tcacgacctc tgagcgcagc ggcatacgcc tcgaggcctg gttcccgcag 300

aactacaccg gccgcttctt gagcactggt aacggtggta tcggaggatg cattcagtat 360

agcgacatcg actatgccgc ctccctcggt ttcgctgccg tcggcgccaa caacggccac 420

gacggcatga ccggcgagcc cttcctcaac aacccggacg ttgtcaccga cttcgcctgg 480

cgctcgctgc acaccggcgt cgtcgtcggc aagcaactca ccgagacctt ctacggcgcg 540

ccccacaaca agtcgtacta cctcggctgc tctactggcg gtcggcaggg cttcaaggcg 600

gtgcaggact ttccggacga cttcgacggc gtcgtcgcgg gcgcgccggc cttcgctttc 660

aatgggctgc tgtactggag cggctcgttc ctgcccgtca ccggaacctc ggaggacccg 720

accttcgtcc ccacggattt ctgggcgcag aatatccacg cggaggtcct ccagcaatgt 780

gacgcgctcg acggcgcgac ggatggggtc ctggagaacc cggacctctg cgacttcgac 840

gtgacgccgc tcatctgcac ggacggcaac acgacgggct gcctgaccga gacgcaggcg 900

gagaccgtgc gcacggtgct cagccccatc tacgacaaga acggcaccct cgtgtacccg 960

cgtctgcagc ctggtgcaga ggtcctcgcc gggtttgccc agttcggcgg ccagcccttc 1020

ctttattcta cggactggta ccgctacgtc gtctacaacg actcgaattg ggaccccatg 1080

acgctctcca ttgacgacta cgccgccgcc cgcgcccaga accccgccaa tgtcgagacg 1140

ttcgagggcg acatctccgc cttcgcgtcc tccggtggca aggtcctgca ctaccacggc 1200

ctgatggacg gcctgatctc ctccgacaac tccaagcggt actacgccct cgttcagcaa 1260

accctcaacc aagagcccgc cgagctcgac gagttctacc gcttcttccc catcagcggg 1320

atgtcgcact gcagcggcgg cgacggcgcg taccgcatcg ggaacgtcga gggcggcgcg 1380

ggcacgagtg cggacgacaa cgtgctgatg tccatggtgc ggtgggtgga ggagggcgtg 1440

gcgccggagg ttgtgcgcgg ggcggacgcg aatgcgactt attggcgcgc gcattgcaag 1500

tggccgaaga cgaacaagta cgtcggaccg ggggattatg aggatgagag tgcatgggag 1560

tgttcttga 1569

<210> 10
<211> 499
<212> PRT
<213> Schizophyllum commune

<220>
<223> ScFae3 without endogenous signal sequence (aa)

<400> 10
Gln Asp Asp Phe Ala Ser Asp Cys Ser Ala Phe Ile Asp Lys Ile Ile
1               5                   10                  15
Leu Asp Asn Val Thr Val Thr Ser Thr Glu Phe Val Ala Ala Gly Thr
            20                  25                  30
Asn Leu Thr Val Tyr Val Pro Asn Ser Cys Gly Ser Pro Ser Tyr Gln
        35                  40                  45
Val Val Ser Thr Asp Leu Cys Arg Ala Thr Met Asn Val Thr Thr Ser
    50                  55                  60
Glu Arg Ser Gly Ile Arg Leu Glu Ala Trp Phe Pro Gln Asn Tyr Thr
65                  70                  75                  80
Gly Arg Phe Leu Ser Thr Gly Asn Gly Gly Ile Gly Gly Cys Ile Gln
                85                  90                  95
Tyr Ser Asp Ile Asp Tyr Ala Ala Ser Leu Gly Phe Ala Ala Val Gly
            100                 105                 110
Ala Asn Asn Gly His Asp Gly Met Thr Gly Glu Pro Phe Leu Asn Asn
        115                 120                 125
Pro Asp Val Val Thr Asp Phe Ala Trp Arg Ser Leu His Thr Gly Val
    130                 135                 140
Val Val Gly Lys Gln Leu Thr Glu Thr Phe Tyr Gly Ala Pro His Asn
145                 150                 155                 160
Lys Ser Tyr Tyr Leu Gly Cys Ser Thr Gly Gly Arg Gln Gly Phe Lys
                165                 170                 175
Ala Val Gln Asp Phe Pro Asp Asp Phe Asp Gly Val Val Ala Gly Ala
            180                 185                 190
Pro Ala Phe Ala Phe Asn Gly Leu Leu Tyr Trp Ser Gly Ser Phe Leu
        195                 200                 205
Pro Val Thr Gly Thr Ser Glu Asp Pro Thr Phe Val Pro Thr Asp Phe
    210                 215                 220
Trp Ala Gln Asn Ile His Ala Glu Val Leu Gln Gln Cys Asp Ala Leu
225                 230                 235                 240
Asp Gly Ala Thr Asp Gly Val Leu Glu Asn Pro Asp Leu Cys Asp Phe

245                         250                         255
Asp Val Thr Pro Leu Ile Cys Thr Asp Gly Asn Thr Thr Gly Cys Leu
            260                         265                         270
Thr Glu Thr Gln Ala Glu Thr Val Arg Thr Val Leu Ser Pro Ile Tyr
        275                         280                         285
Asp Lys Asn Gly Thr Leu Val Tyr Pro Arg Leu Gln Pro Gly Ala Glu
    290                         295                         300
Val Leu Ala Gly Phe Ala Gln Phe Gly Gly Gln Pro Phe Leu Tyr Ser
305                         310                         315                         320
Thr Asp Trp Tyr Arg Tyr Val Val Tyr Asn Asp Ser Asn Trp Asp Pro
                        325                         330                         335
Met Thr Leu Ser Ile Asp Asp Tyr Ala Ala Ala Arg Ala Gln Asn Pro
                340                         345                         350
Ala Asn Val Glu Thr Phe Glu Gly Asp Ile Ser Ala Phe Ala Ser Ser
        355                         360                         365
Gly Gly Lys Val Leu His Tyr His Gly Leu Met Asp Gly Leu Ile Ser
    370                         375                         380
Ser Asp Asn Ser Lys Arg Tyr Tyr Ala Leu Val Gln Gln Thr Leu Asn
385                         390                         395                         400
Gln Glu Pro Ala Glu Leu Asp Glu Phe Tyr Arg Phe Phe Pro Ile Ser
                        405                         410                         415
Gly Met Ser His Cys Ser Gly Gly Asp Gly Ala Tyr Arg Ile Gly Asn
                420                         425                         430
Val Glu Gly Gly Ala Gly Thr Ser Ala Asp Asp Asn Val Leu Met Ser
        435                         440                         445
Met Val Arg Trp Val Glu Glu Gly Val Ala Pro Glu Val Val Arg Gly
    450                         455                         460
Ala Asp Ala Asn Ala Thr Tyr Trp Arg Ala His Cys Lys Trp Pro Lys
465                         470                         475                         480
Thr Asn Lys Tyr Val Gly Pro Gly Asp Tyr Glu Asp Glu Ser Ala Trp
                        485                         490                         495
Glu Cys Ser

<210> 11
<211> 23
<212> PRT
<213> Schizophyllum commune

<220>
<223> Endogenous signal sequence ScFae3 (aa)

<400> 11
Met Ala Pro Leu Arg Ser Val Ser Ser Val Leu Val Gly Ala Leu Ala
1               5                   10                  15
Leu Leu Arg Ala Ala His Ala
            20

<210> 12
<211> 522
<212> PRT
<213> Schizophyllum commune

<220>
<223> ScFae3 with endogenous signal sequence (aa)

<400> 12
Met Ala Pro Leu Arg Ser Val Ser Ser Val Leu Val Gly Ala Leu Ala
1               5                   10                  15
Leu Leu Arg Ala Ala His Ala Gln Asp Asp Phe Ala Ser Asp Cys Ser
            20                  25                  30

Ala Phe Ile Asp Lys Ile Ile Leu Asp Asn Val Thr Val Thr Ser Thr
        35                  40                  45
Glu Phe Val Ala Ala Gly Thr Asn Leu Thr Val Tyr Val Pro Asn Ser
    50                  55                  60
Cys Gly Ser Pro Ser Tyr Gln Val Val Ser Thr Asp Leu Cys Arg Ala
65                  70                  75                  80
Thr Met Asn Val Thr Thr Ser Glu Arg Ser Gly Ile Arg Leu Glu Ala
                85                  90                  95
Trp Phe Pro Gln Asn Tyr Thr Gly Arg Phe Leu Ser Thr Gly Asn Gly
            100                 105                 110
Gly Ile Gly Gly Cys Ile Gln Tyr Ser Asp Ile Asp Tyr Ala Ala Ser
        115                 120                 125
Leu Gly Phe Ala Ala Val Gly Ala Asn Asn Gly His Asp Gly Met Thr
    130                 135                 140
Gly Glu Pro Phe Leu Asn Asn Pro Asp Val Val Thr Asp Phe Ala Trp
145                 150                 155                 160
Arg Ser Leu His Thr Gly Val Val Val Gly Lys Gln Leu Thr Glu Thr
                165                 170                 175
Phe Tyr Gly Ala Pro His Asn Lys Ser Tyr Tyr Leu Gly Cys Ser Thr
            180                 185                 190
Gly Gly Arg Gln Gly Phe Lys Ala Val Gln Asp Phe Pro Asp Asp Phe
        195                 200                 205
Asp Gly Val Val Ala Gly Ala Pro Ala Phe Ala Phe Asn Gly Leu Leu
    210                 215                 220
Tyr Trp Ser Gly Ser Phe Leu Pro Val Thr Gly Thr Ser Glu Asp Pro
225                 230                 235                 240
Thr Phe Val Pro Thr Asp Phe Trp Ala Gln Asn Ile His Ala Glu Val
                245                 250                 255
Leu Gln Gln Cys Asp Ala Leu Asp Gly Ala Thr Asp Gly Val Leu Glu
            260                 265                 270
Asn Pro Asp Leu Cys Asp Phe Asp Val Thr Pro Leu Ile Cys Thr Asp
        275                 280                 285
Gly Asn Thr Thr Gly Cys Leu Thr Glu Thr Gln Ala Glu Thr Val Arg
    290                 295                 300
Thr Val Leu Ser Pro Ile Tyr Asp Lys Asn Gly Thr Leu Val Tyr Pro
305                 310                 315                 320
Arg Leu Gln Pro Gly Ala Glu Val Leu Ala Gly Phe Ala Gln Phe Gly
                325                 330                 335
Gly Gln Pro Phe Leu Tyr Ser Thr Asp Trp Tyr Arg Tyr Val Val Tyr
            340                 345                 350
Asn Asp Ser Asn Trp Asp Pro Met Thr Leu Ser Ile Asp Asp Tyr Ala
        355                 360                 365
Ala Ala Arg Ala Gln Asn Pro Ala Asn Val Glu Thr Phe Glu Gly Asp
    370                 375                 380
Ile Ser Ala Phe Ala Ser Ser Gly Gly Lys Val Leu His Tyr His Gly
385                 390                 395                 400
Leu Met Asp Gly Leu Ile Ser Ser Asp Asn Ser Lys Arg Tyr Tyr Ala
                405                 410                 415
Leu Val Gln Gln Thr Leu Asn Gln Glu Pro Ala Glu Leu Asp Glu Phe
            420                 425                 430
Tyr Arg Phe Phe Pro Ile Ser Gly Met Ser His Cys Ser Gly Gly Asp
        435                 440                 445
Gly Ala Tyr Arg Ile Gly Asn Val Glu Gly Gly Ala Gly Thr Ser Ala
    450                 455                 460
Asp Asp Asn Val Leu Met Ser Met Val Arg Trp Val Glu Glu Gly Val
465                 470                 475                 480
Ala Pro Glu Val Val Arg Gly Ala Asp Ala Asn Ala Thr Tyr Trp Arg
                485                 490                 495
Ala His Cys Lys Trp Pro Lys Thr Asn Lys Tyr Val Gly Pro Gly Asp
            500                 505                 510
Tyr Glu Asp Glu Ser Ala Trp Glu Cys Ser
        515                 520

<210> 13
<211> 7
<212> PRT
<213> Artificial Sequence

<220>
<223> N-terminal heterologous amino acid sequence of expressed ScFae
      (aa)

<400> 13
Glu Ala Glu Ala Tyr Val Glu
1               5

## Description

The present invention relates to the use of enzymes in the production of food. In particular, the invention provides novel methods and applications for enzymes for the exploitation of agro-industrial by-products to obtain more palatable feeds, in particular food products suitable for human consumption, but also animal feeds. More specifically, the invention discloses a method for enzymatically degrading kaempferol-3-*O*-(*2"'*-*O*-sinapoyl-β-sophoroside), a recently identified bitter-constituent present in rapeseed that has, so far, prevented this plant from serving as a protein source for human nutrition. The invention further teaches the use of enzymes derived from the basidiomycete *Schizophyllum commune* that are capable of hydrolyzing sinapyl ester-containing kaempferol-3-*O*-*(2"'*-*O*-sinapoyl-β-sophoroside) into non-bitter degradation products. Finally, the invention provides a feed, e.g., a food product for human consumption or an animal feed obtainable by the methods disclosed herein.

Since ancient times, humans have produced vegetable oils from a broad variety of plant seeds and fruits. Today, soybean (351.3 million tons), rapeseed/canola (71.3 million tons), sunflower (47.8 million tons), peanut (43.1 million tons) and cottonseed (39.1 million tons) represent the five most cultivated oil seed crops in the world (Hald *et al.,* 2019). During the production of vegetable oils, millions of tons of agro-industrial by-products are generated. These by-products are usually rich in protein and thus have the potential to meet the rapidly increasing global food demand, especially, since protein has been identified as a limiting macronutrient in human nutrition. This may also contribute to global food security (Hald *et al*, 2019). The majority of these highly nutritious by-products are nowadays spent as animal feed or fertilizer (Nieter *et al.,* 2016a). This is largely due to the presence of accompanying substances that restrict the palatability and salubriousness of such by-products not only for humans but also many domesticated animals. Facing these obstacles, several genetically modified crop variants have been developed in the past with the aim to reduce the levels of anti-nutritional factors in animal feed produced from these crops. Many of these crop varieties are presently grown by farmers around the globe. However, the increasing concerns of large parts of Western society towards genetically modified crops necessitates the development of alternative strategies.

By-products obtainable during rapeseed oil extraction exhibit a particularly well-balanced amino acid composition with a high nutritional value that, if properly harnessed, could make significant contributions to the on-going long-term development of replacing animal proteins with those derived from plant sources. However, no commercial food products on the basis of rapeseed are currently available for human consumption, primarily due to its strong bitter off-taste. A series of compounds present in rapeseed have been previously suspected of contributing to this bitterness, including glucosinolates and phenols. However, in a recently published study, taste-guided fractionation and mass spectrometric analyses of rapeseed protein isolates resulted in the identification of kaempferol 3-*O*-(*2"'*-*O*-sinapoyl-β-sophoroside) as the key compound responsible for the unpleasant bitter taste of this plant (Hald *et al.,* 2019). Although it has not been thoroughly investigated yet, it is likely that kaempferol 3-*O*-*(2"'*-*O*-sinapoyl-β-sophoroside) may also contribute to the bitterness of other crop seeds.

Kaempferol-3-*O*-(2"'-*O*-sinapoyl-β-sophoroside) is an ester formed by sinapinic acid and a kaempferol-sophoroside moiety. Kaempferol is a naturally occurring flavonol that belongs to the class of secondary polyphenolic metabolites of plants and fungi collectively known as flavonoids. Sophorose is a glucose-derived disaccharide consisting of two monosaccharides connected via a β-1,2 glycosidic bond.

Given the very recent identification of kaempferol 3-*O*-*(2"'*-*O*-sinapoyl-β-sophoroside) as a bitter substance, there is a lack of available technological processes or breeding strategies that effectively eliminate this compound from rapeseed-derived agro-industrial by-products. One possible approach represents the use of enzymes that can specifically degrade kaempferol 3-*O*-*(2"'*-*O*-sinapoyl-β-sophoroside) at high efficiency. The application of enzymes to improve quality and palatability of food is well known. For example, the stomach-irritating chlorogenic acids in coffee were rapidly and selectively removed by an immobilized esterase from the basidiomycete *Rhizoctonia solanii* (Siebert *et al.* 2019). However, so far, no enzymes capable of degrading kaempferol 3-*O*-*(2"'*-*O*-sinapoyl-β-sophoroside) have been identified.

Hence, in the light of the state of the art, there exists an urgent need for effective methods capable of removing bitter compounds such as kaempferol 3-*O*-(*2"'*-*O*-sinapoyl-β-sophoroside) from natural materials and/or agro-industrial by-products to secure new sources of food for both humans and animals.

This problem is solved by the invention, especially by the subject matter of the claims.

The present invention provides a method of preparing a feed, e.g., an animal feed or food product for human consumption, comprising contacting an agro-industrial by-product with an enzyme capable of hydrolysing the ester bond in kaempferol-3-*O*-*(2"'*-*O*-sinapoyl-β-sophoroside) and comprising an amino acid sequence having at least 80% amino acid sequence identity to SEQ ID NO: 1.Thus, in the method of the invention kaempferol-3-*O*-*(2"'-O*-sinapoyl-β-sophoroside) is hydrolyzed to non-bitter components.

Preferably, the levels of kaempferol-3-*O*-*(2"'*-*O*-sinapoyl-β-sophoroside) in the feed or food product are decreased by at least 20%, more preferably by at least 25%, at least 30%, at least 35%, at least 40%, at least 45%, at least 50%, at least 55%, at least 60% or at least 65% compared to the levels found in the agro-industrial by-product prior to being subjected to the method of the invention. In a particularly preferred embodiment, the level of kaempferol 3-*O*-*(2"'*-*O*-sinapoyl-β-sophoroside) is decreased below the level that, on average, can be tasted by humans, which has been determined to be 3.4 µmol/L. Thus, preferably, the level of kaempferol 3-*O*-*(2"'*-*O*-sinapoyl-β-sophoroside) is reduced to less than 3.4 µmol/L, preferably, less than 1.7 µml/L, which has been determined to be the lowest level detectable by human test persons (Hald *et al.,* 2019).

In the context of the invention, an agro-industrial by-product relates to a material that is generated as a secondary product during industrial and/or agricultural production processes. It is generated during production of a first product, such as a vegetable oil. Agro-industrial by-products according to the invention are preferably natural materials that arise during the processing of plants, such as crops, fruits or vegetables. For instance, a by-product may be a press cake obtainable from vegetable oil production, bran obtainable from cereal grain processing, brewer's spent grain obtainable from beer processing, pomace obtainable from grape processing, peel obtainable from potato or carrot processing, leaves obtainable from salad processing or shells obtainable from nut processing. These by-products may be considered waste products, i.e. they may not be intended for further processing and thus be discarded. However, the majority of agro-industrial by-products serve important functions themselves, e.g., as feed for domestic livestock, as fertilizers or as substrates in biogas or bioethanol plants. The method of the invention provides a higher quality feed, because of reduced bitterness. It can thus be used in a higher percentage of the total feed, or it is tastier.

In a preferred embodiment, the agro-industrial by-product is obtainable during the production of rapeseed oil. In the context of the present invention, rapeseed refers to seeds of the genus *Brassica* (*Brassica napus* or *Brassica juncea*). Preferably, it is canola, in particular, if the feed obtained is food for human consumption. Canola comprises a reduced content of the toxic erucic acid. The content of erucic acid has been reduced through long-term breeding efforts to levels suitable for animal or, preferably, human consumption. This may also be effected by other methods. Rapeseed oil represents the third most consumed vegetable oil worldwide in 2018/2019 (US Departments of Agriculture and USDA Foreign Agricultural Service 2019).

Vegetable oil may be prepared by means of mechanical extraction, e.g. via expeller-pressing, screw-pressing, ram-pressing or through milling using mortar and pestle. The resulting by-product is referred to as a seed meal press cake. Mechanical extraction may be performed with seeds that have been previously conditioned with heat. Alternatively, vegetable oils can be cold-extracted, at temperatures not higher than 60°C, to obtain so-called cold-pressed seed meal press cakes. Mechanical cold extraction of vegetable oils represents the most preferred extraction method amongst Western costumers that prefer natural, healthy food products. Vegetable oil extraction from oil seeds or fruits may alternatively or simultaneously be achieved via use of a suitable chemical solvent to further maximize oil yield. The most commonly utilized solvent for vegetable oil extraction is hexane. Hexane is mostly used during production of soybean or corn oils. Before performing solvent extraction, oil seeds need to be cracked or ground (Kumar *et al.,* 2017). Importantly, the solvent has to be completely removed from the by-product that is to be further processed, e.g. via evaporation at temperatures of about 150°C or higher. Alternatively or additionally, non-toxic, "green" tools and methods may be used for oil extraction, such as supercritical carbon dioxide, different enzymes or terpenes. Collectively, the agro-industrial by-product of the invention may therefore be a solid or semi-solid leftover from vegetable oil extraction, preferably a seed meal press cake, most preferably a rapeseed meal press cake.

The agro-industrial by-product used for the invention comprises the chemical substance kaempferol-3-*O*-(2*"'*-*O*-sinapoyl-β-sophoroside).

As described above, kaempferol-3-*O*-*(2"'*-*O*-sinapoyl-β-sophoroside) has been recently identified as the key bitter compound in protein isolates of rapeseed. In addition, kaempferol-3-*O*-*(2"'*-*O*-sinapoyl-β-sophoroside) is found in tronchuda cabbage (*B*. *oleracea* L. *var. costata* DC) and transgenic low-sinapine oilseed rape. Other glycosides of kaempferol occur in ferns, conifers and flowering plants, including many fruits and vegetables (Calderón-Montano *et al.,* 2011). Rapeseed alone comprises 39 kaempferol derivates, mostly glycosides (Shao *et al.,* 2014). Kaempferol itself is a naturally occurring flavonol present in a wide variety of foods such as apples, grapes, tomatoes, green tea, potatoes, onions, broccoli, Brussels sprouts, squash, cucumbers, lettuce, green beans, peaches, blackberries, raspberries, and spinach as well as many additional plants suitable for human consumption. Furthermore, kaempferol has been identified in plants commonly used for the production of vegetable oils such as rapeseed (*Brassica napus*), soybean (*Glycine max*) or ben oil trees (*Moringa oleifera*). The known bitter taste of several of these plants may suggest that the kaempferol is provided in the form of kaempferol-3-*O*-*(2"'*-*O*-sinapoyl-β-sophoroside) or other, closely related kaempferol glucosides/sophorosides that might cause bitter off-tastes. Therefore, the herein disclosed method may also improve the palatability of foods from sources other than rapeseed.

The inventors considered the use of an enzyme to degrade the bitter compounds, in particular, kaempferol 3-*O*-*(2"'*-*O*-sinapoyl-β-sophoroside). According to its chemical structure, kaempferol 3-*O*-*(2"'*-*O*-sinapoyl-β-sophoroside) may possible be degradable by a glucosidase or esterase. Glucosidases may cleave the glucoside bond of the sophoroside moiety, whereas esterases may hydrolyse the ester linkage between the sophoroside moiety and the sinapinic acid. Both options would result in non-bitter products (Hald *et al.* 2019). The inventors tested different feruloyl esterases with regard to their capability to degrade kaempferol 3-*O*-*(2"'*-*O*-sinapoyl-β-sophoroside), SwFae isolated from *Streptomyces werraensis* (Schulz *et al.,* 2018), PeFae from *Pleurotus eryngii* (Nieter *et al.,* 2014), and ScFae1 from *Schizophyllum commune* (WO 2016 026793 A1).

Feruloyl esterases (FAEs) are defined by their capability for hydrolyzing the ester linkage between polysaccharides and ferulic acid and related hydroxycinnamic acids such as cinnamic acid, caffeic acid, chlorogenic acid, sinapinic acid, coumaric acid or chicoric acid. Crepin *et al.* (2004) developed a classification system for FAEs, dividing them into four types (A-D) based on their substrate specificity supported by primary sequence identity. Type A FAEs are active on methyl ferulate, methyl-p-coumarate, methyl sinapate but not on methyl caffeate. In contrast, Type B FAEs hydrolyze methyl ferulate, methyl-p-coumarate and methyl caffeate, while Type C and D act on all four substrates. The above mentioned feruloyl esterases SwFae and ScFae1 are both classified as type D FAEs, whereas PeFae belongs to the group of type A esterases.

Table 1 below shows that SwFae, and PeFae have relatively high activities towards methyl sinapate, the substrate with previously known cleavage activities most related to kaempferol 3-*O*-*(2"'*-*O*-sinapoyl-β-sophoroside), and ScFae1 has a relatively low substrate specificity towards methyl sinapate (Nieter *et al.,* 2016a).

However, the inventors surprisingly found that, in contrast to the two feruloyl esterases having a relatively high activity towards methyl sinapate, the feruloyl esterase ScFae1 could effectively catalyze the hydrolysis of kaempferol-3-*O*-*(2"'*-*O*-sinapoyl-β-sophoroside) into the non-bitter products sinapinic acid and a sophoroside moiety.

In addition, ScFae1 cleaved another uncharacterized sophoroside derivative of kaempferol as well as a kaempferol glycoside identified as kaempferol-3-O-sinapylglucoside-7-O-glucoside (Example below). In contrast, the recombinant feruloyl esterase SwFae isolated from *Streptomyces werraensis* exhibited a considerably better cleavage activity towards methyl sinapate, but showed no detectable hydrolytic activity towards the sinapyl ester linkage of the bitter kaempferol sophoroside. Similarily, the feruloyl esterase from *Pleurotus eryngii* (PeFae) that also possessed a better relative activity towards methyl sinapate than ScFae1, did not degrade kaempferol 3-*O*-*(2"'*-*O*-sinapoyl-β-sophoroside). Therefore, the inventors arrived at the unexpected conclusion that a sinapyl esterase activity towards methyl sinapate does not provide any indication of the capability of an enzyme to hydrolyze kaempferol 3-*O*-*(2"'*-*O*-sinapoyl-β-sophoroside). The enzyme used in the method according to the invention thus advantageously has a high substrate specificity for kaempferol glycosides/sophorosides.

The enzyme of the invention preferably comprises a sequence having SEQ ID NO: 4, and is designated ScFae1. The enzyme may also comprise an amino acid sequence having at least 80%, at least 85%, at least 90%, at least 95%, at least 96%, at least 97%, at least 98%, or at least 99% amino acid identity to SEQ ID NO: 4, wherein the enzyme is also capable of hydrolyzing kaempferol-3-*O*-*(2"'*-*O*-sinapoyl-β-sophoroside) e.g., as defined herein. Said enzyme may, e.g., be another *S. commune*-derived feruloyl esterase such as ScFaeD2 which has 82% amino acid sequence identity to ScFae1 (Nieter et al., 2016). It may also consist of SEQ ID NO: 4. In some embodiments, the enzyme comprising SEQ ID NO: 4 does not comprise the endogenous signaling sequence of SEQ ID NO: 5. It may instead comprise a heterologous signaling sequence to improve expression. For instance, in Nieter et al., 2016, the enzyme was produced heterologously in *Pichia pastoris* using the pPIC9 vector. Accordingly, the intrinsic alpha factor of *Saccharomyces cerevisiae* was attached as a signaling sequence so that extracellular formation could take place. The alpha factor was subsequently cleaved off during processing, but a short stretch of amino acids was retained behind the junction at the N-terminus of the enzyme. When cloning with EcoRI the retained stretch of amino acid residues consisted of EAEAYVE (SEQ ID NO: 13). The enzyme may however also comprise no signaling sequence after processing. In other embodiments, it has the full length sequence of ScFae1 including the endogenous signaling sequence (SEQ ID NO: 6). ScFae1 may be encoded by a nucleic acid comprising SEQ ID NO: 1.

In another embodiment, the enzyme may comprise a sequence having SEQ ID NO: 10, and is designated ScFae3. It may also consist of SEQ ID NO: 10. In some embodiments, the enzyme comprising SEQ ID NO: 10 does not comprise the endogenous signaling sequence of SEQ ID NO: 11. It may instead comprise a heterologous signaling sequence to improve expression, e.g., as specified herein, or, after processing, no signaling sequence. In other embodiments, it is the full length sequence of ScFae3 provided by the inventors (SEQ ID NO: 12). ScFae3 may be encoded by a nucleic acid comprising SEQ ID NO: 7.

The catalytic activity of the enzyme of the invention should be sufficient to decrease the levels of kaempferol 3-*O*-*(2"'*-*O*-sinapoyl-β-sophoroside) in the agro-industrial by-product or in a purified sample by at least 20%, preferably by at least 25%, at least 30%, at least 35%, at least 40%, at least 45%, at least 50%, at least 55%, at least 60% or at least 65%. In a particularly preferred embodiment, the level of kaempferol 3-O-(2"'-O-sinapoyl-β-sophoroside) is decreased below the level of taste recognition by humans, as defined herein. Accordingly, the enzyme of the invention capable of hydrolyzing kaempferol 3-*O*-*(2"'*-*O-*sinapoyl-β-sophoroside) may have at least 30%, at least 33.3%, at least 40%, at least 50%, at least 60%, at least 70%, at least 80%, at least 90% or at least 100% of the respective activity determined for ScFae1 described in the example below. It may also have a higher activity.

In addition to specifically cleaving kaempferol glycosides, earlier tests revealed that the type C and D FAEs ScFae1 and ScFae3 are capable of hydrolyzing both methyl ferulate as well as methyl caffeate at high efficiency. Furthermore, ScFae1 and ScFae3 were previously found to further catalyze the cleavage of chlorogenate esters formed by caffeic acid and quinic acid. ScFae1 and ScFae3 thus represent enzymes with an exceptionally broad substrate specificity making them suitable for a wide variety of industrial applications. Therefore, in addition to having kaempferol 3-*O*-*(2"'*-*O*-sinapoyl-β-sophoroside) esterase activity, the enzyme used in the method according to the invention may further exhibit feruloyl esterase activity, e.g., type C and D feruloyl esterase activity, and/or chlorogenic acid esterase activity.

The enzyme of the invention may further have one or more of the following characteristics, preferably, all characteristics listed under a) to f):
a) glycosylation;
b) feruloyl acid esterase activity at about pH 3-9.5;
c) pH optimum of feruloyl acid esterase activity at about pH 4.5-9.5, most preferably at about pH 5-8.5;
d) type C and D feruloyl esterase activity on the substrates methyl p-coumarate, methyl ferulate, methyl caffeate, and methyl sinapate;
e) thermal stability up to about 50°C;
f) optimal activity at about 30-50°C, preferably about 39-43°C; and /or
comprising a tag suitable for purification, such as a His tag, preferably, at the C-terminus.

In a preferred embodiment, the enzyme may be obtainable from a basidiomycete, preferably from the *Schizophyllum* genus, most preferably from *Schizophyllum commune,* and have at least 80% sequence identity with ScFae1. Homologues from other basidiomycetes may be obtainable for the skilled person, e.g., comprising using a probe from the nucleotide sequence encoding the enzyme of the invention for screening a cDNA bank of the basidiomycete under low stringency, medium stringency or high stringency conditions, thus isolating a homologous nucleic acid, and expressing the same in a suitable host cell. Alternatively, purification methods such as a combination of chromatographic steps, e.g., anion exchange chromatography on Q Sepharose FF, preparative IEF, and a combination of hydrophobic interaction chromatography and affinity chromatography, such as Nickel-NTA-agarose gel affinity chromatography or polyamide affinity chromatography, can be employed to purify homologous enzymes from other basidiomycetes.

The enzyme may be obtainable by recombinant expression in a suitable heterologous host cell, e.g., in yeast, preferably in *Saccharomyces, Pichia, Kluyveromyces, Hansenula* and *Yarrowia* genera, more preferably in *Kluyveromyces lactis, Saccharomyces cerevisiae,* or *Schizosaccharomyces pombe,* and most preferably in *Pichia pastoris,* e.g., *P. pastoris* SMD 1168, herein also referred to by its official name as *Komagataella phaffii.*

The invention also provides a method of hydrolyzing an ester of kaempferol-3-*O*-*(2"'*-*O-*sinapoyl-β-sophoroside), the method comprising contacting kaempferol-3-O-*(2"'*-*O-*sinapoyl-β-sophoroside) with an enzyme capable of hydrolyzing the ester bond in kaempferol 3-*O*-*(2"'*-*O*-sinapoyl-β-sophoroside) and comprising an amino acid sequence having at least 80% amino acid sequence identity to SEQ ID NO: 1 or to SEQ ID NO: 4.

The kaempferol-3-*O*-*(2"'*-*O*-sinapoyl-β-sophoroside) may be present in a composition, e.g., protein isolate, derived from plant seeds, preferable from rapeseed. Alternatively, the kaempferol-3-*O*-*(2"'*-*O*-sinapoyl-β-sophoroside) may have been fully purified from its source prior to being contacted with the enzyme of the invention. In a preferred embodiment, the kaempferol-3-*O*-*(2"'*-*O*-sinapoyl-β-sophoroside) is present in an agro-industrial by-product obtainable during vegetable oil production and is hydrolyzed to reduce the bitter off-taste of said by-product. Notably, the method of the invention may also reduce the levels of kaempferol-3-*O*-*(2"'*-*O*-sinapoyl-β-sophoroside) in plant seeds, fruits as well as any type of plant or diluted homogenate thereof that have not been otherwise processed, e.g., for oil extraction.

According to the herein described methods, the agro-industrial by-product may be directly contacted with the enzyme of the invention to induce the degradation of kaempferol-*3*-*O-(2"'*-*O*-sinapoyl-β-sophoroside). In a preferred embodiment, the by-product is first diluted and homogenized in a liquid suitable for animal as well as human consumption (e.g., water) prior to being contacted with the enzyme of the invention. Dilution may occur, e.g., in a ratio of 1:1 - 1:5, e.g., 1:1, 1:2, 1:3, 1:4, or 1:5 in the diluent. The enzymatic reaction may be performed under suitable conditions to maximize the enzymatic hydrolysis of kaempferol-3-*O-(2"'*-*O*-sinapoyl-β-sophoroside). Suitable conditions can be determined by the skilled person or are described in the example below, e.g., 37°C, pH 7.5. After completion of the catalyzed reaction, the diluted and homogenized agro-industrial by-product may be concentrated or dried prior to further processing. Alternatively, the diluted homogenate is directly used for its intended purpose. For example, a food product for human consumption may be prepared on its basis.

The invention further provides a method of preparing a product selected from the group comprising
a. sinapinic acid; and/or
b. a kaempferol sophoroside moiety;
the method comprising contacting the educts of the catalyzed reaction generating the respective product under suitable conditions with an enzyme capable of hydrolyzing the ester bond in kaempferol 3-*O*-*(2"'*-*O*-sinapoyl-β-sophoroside) and comprising an amino acid sequence having at least 80% amino acid sequence identity to SEQ ID NO: 1, wherein the educt is a kaempferol sophoroside, preferably kaempferol-3-*O*-*(2"'*-*O*-sinapoyl-β-sophoroside) that may be present in purified form, inside a sample of a natural material (e.g., a protein isolate from a plant) or, preferably, in an agro-industrial by-product according to the invention, e.g. a rapeseed press cake.

The products of a. and b. are products of the hydrolysis reactions catalyzed by the enzyme of the invention.

The invention also provides the use of an enzyme capable of hydrolyzing the ester bond in kaempferol-3-*O*-*(2"'*-*O*-sinapoyl-β-sophoroside) and an amino acid sequence having at least 80% amino acid identity to SEQ ID NO: 1 in a method for reducing the bitterness of an agro-industrial by-product.

In a preferred embodiment, said use of the enzyme includes the implementation of any of the herein disclosed methods.

One object of the invention thus also is a feed obtainable from the method of the invention. The term "feed" is understood to refer to agricultural foodstuff or forage used to feed animals, e.g., domesticated livestock, or to human food.

Said domesticated livestock may comprise animals selected from the group comprising cattle, sheep, pigs, horses, goats, camels, chickens, turkeys, geese, ducks or rodents. The animals may also be fish. The feed may be provided in dried form, e.g. as dried flakes, milled powder or compressed pellets. Alternatively, the feed may be supplied in a liquid or semi-liquid form. For instance, the feed may be diluted and homogenized in a liquid suitable for animal consumption such as water or milk. In one embodiment, the feed consists entirely of the product obtainable through the method of the invention. In another embodiment, the feed of the invention further mixed in a compound animal feed that additionally comprises materials such as hay, straw, silage, various feed grains, household food scraps, animal meal or fish meal. The feed may further be supplemented with additional vitamins, minerals, trace elements, chemical preservatives, antibiotics or fermentation products.

In a preferred embodiment, the method of the invention provides a food product or food ingredient suitable for human consumption. This adds an easily available protein-rich component to the human diet. The food product or ingredient may, for example, be an ingredient of an instant, ready-made food mix. It may also be provided in the form of a powder, pellet or stock cube that can be added to food during cooking, e.g. by dissolving it in boiling water used for the preparation of a soup, sauce or stew. The food product may also be a nutritious dietary supplement that may be consumed with or in between regular meals. For example, the food product may be dissolved or suspended in drinks such as water, juices or animal- or plant-milk to facilitate its consumption. It may thus serve as a protein supplement for people with limited access to high quality protein sources, but also for vegetarians or vegans, or amateur or professional athletes. The food may also be provided in a form that can be directly consumed by a human, for instance, as an ingredient of cereal mixes, muesli-bars, chocolates, snacks, protein enriched bakery products, meat substitutes, vegetarian spreads or coffee whitener. It may also be used as a replacement for protein from genetically modified soy.

Accordingly, the invention discloses a feed, e.g., an animal feed or food product, obtainable by the method of the invention that comprises an enzyme selected from the group comprising an enzyme having at least 80% amino acid identity to SEQ ID NO: 1 wherein said enzyme is optionally capable of hydrolyzing the ester bond in kaempferol 3-*O*-*(2"'*-*O-*sinapoyl-β-sophoroside). When the hydrolysis is sufficient the enzyme may be deactivated, e.g., denatured, so that it is not any more functional.

The final feed product may comprise, e.g., 1-99 % vegetable meal press cake, obtainable from the production of vegetable oil and from the method of the invention, preferably, rapeseed meal press cake, or, more preferably, canola seed meal press cake, such as 5-90%, 10-80%, 20-70%, 30-60% or 40-50%. If not mentioned otherwise, herein, % refers to weight/weight.

The feed, e.g., food product, obtainable by the method according to the invention is characterized by decreased levels of the bitter-compound kaempferol-3-*O*-*(2"'*-*O*-sinapoyl-β-sophoroside). It may also comprise reduced levels of other sophoroside derivatives of kaempferol.

Preferably, the levels of kaempferol-3-*O*-*(2"'*-*O*-sinapoyl-β-sophoroside) in the feed or food product are decreased by at least 20%, more preferably by at least 25%, at least 30%, at least 35%, at least 40%, at least 45%, at least 50%, at least 55%, at least 60% or at least 65% compared to the levels found in the agro-industrial by-product prior to being subjected to the method of the invention. In a particularly preferred embodiment, the level of kaempferol 3-*O*-*(2"'*-*O*-sinapoyl-β-sophoroside) is decreased below the level that, on average, can be tasted by humans, which has been determined to be 3.4 µmol/L. Thus, preferably, the feed, e.g., food product for human consumption, contains less than 3.4 µmol/L kaempferol 3-*O*-*(2"'*-*O*-sinapoyl-β-sophoroside), preferably, less than 1.7 µml/L, which has been determined to be the lowest level detectable by human test persons (Hald *et al.,* 2019). Further, preferably, the feed, e.g., food product for human consumption does not comprise levels of erucic acid or other fatty acid structurally related thereto that are not recognized as safe for humans, i.e., less than 5%, preferably, less than 2% erucic acid. Furthermore, the food product preferably does not comprise thioglucosides such as 3-butenylglucosinolate, 4- pentenylglucosinolate, 2-hydroxy-3-butenylglucosinolate or 2-hydroxy-4-pentenylglucosinolate. None of these are present in modern rapeseed lines.

While the agro-industrial by-products used as a basis for the method of the invention may sometimes also be used as animal feed, the method of the invention provides a higher quality feed, because of reduced bitterness. It can thus be used in a higher percentage of the total feed, or is tastier, and consequently more of the feed is consumed. Thus, if used for feeding livestock, e.g., cattle, in one embodiment, the invention provides a method of cultivating livestock, comprising contacting an agro-industrial by-product with an enzyme having feruloyl esterase activity and comprising an amino acid sequence having at least 80% amino acid sequence identity to SEQ ID NO: 1 to obtain a feed, and feeding said livestock with the feed. As the bitterness of the protein rich agro-industrial by-product is decreased, the livestock will consume more of the feed. Thus, they have a higher intake of protein from this agro-industrial by-product than with non-treated product. Optionally, the feed of the present invention can thus partly or completely substitute other protein-rich feed components which may be more expensive.

In summary, the invention teaches the use of enzymes capable of hydrolyzing the ester bond in kaempferol-3-*O*-*(2"'*-*O*-sinapoyl-β-sophoroside) with specified amino acid sequences in a method for preparing an animal feed, e.g., a food product suitable for human consumption, from an agro-industrial by-product comprising the bitter compound kaempferol-3-*O*-*(2"'*-*O*-sinapoyl-β-sophoroside). As disclosed herein, these enzymes are unexpectedly capable of hydrolyzing the ester bond of bitter-tasting kaempferol sophorosides to catalyze the production on non-bitter reaction products. As a result, the agro-industrial by-product exhibits a reduced bitter off-taste and can be utilized as a more palatable animal feed and, in particular, may for the first time be made available for human consumption.

Throughout the invention, the term "about" is intended to be understood as "+/- 10%". If "about" relates to a range, it refers to both lower and upper limit of the range. "A" is intended to mean "one or more", if not explicitly mentioned otherwise.

All literature cited herein is herewith fully incorporated. The present invention is further illustrated, but not limited, by the following example.

### Brief description of the Drawings

**Fig. 1****:** LC-MS/MS chromatogram (scan m/z 815). Control sample (upper curve) and enzymatically treated sample (lower curve) (A). Peak 1 was identified as the key bitter compound kaempferol 3-*O*-*(2"'*-*O*-sinapoyl-*β*-sophoroside) (B).
**Fig. 2****:** Mass spectrum of peak 1 (A) in comparison with reference spectrum according to Hald *et al.* 2019 (B).
**Fig. 3****:** LC-MS/MS chromatogram (scan m/z 223). Sinapinic acid concentration of the enzymatically treated sample (upper curve) and the untreated control sample (lower curve).

SEQ ID NO: 1 ScFae1 without endogenous signal sequence (na)
SEQ ID NO: 2 Endogenous signal sequence ScFae1 (na)
SEQ ID NO: 3 ScFae1 with endogenous signal sequence (na)
SEQ ID NO: 4 ScFae1 without endogenous signal sequence (aa)
SEQ ID NO: 5 Endogenous signal sequence ScFae1 (aa)
SEQ ID NO: 6 ScFae1 with endogenous signal sequence (aa)
SEQ ID NO: 7 ScFae3 without endogenous signal sequence (na)
SEQ ID NO: 8 Endogenous signal sequence ScFae3 (na)
SEQ ID NO: 9 ScFae3 with endogenous signal sequence (na)
SEQ ID NO: 10 ScFae3 without endogenous signal sequence (aa)
SEQ ID NO: 11 Endogenous signal sequence ScFae3 (aa)
SEQ ID NO: 12 ScFae3 with endogenous signal sequence (aa)
SEQ ID NO: 13 N-terminal heterologous amino acid sequence of expressed ScFae (aa)

### EXAMPLE

In recent years, various esterases capable of hydrolysing methyl sinapate were identified, produced in pure form through heterologous hosts, and characterized. A feruloyl esterase from *Streptomyces werraensis* (SwFAE) (Schulz *et al.* 2018) and one from *Pleurotus eryngii* (PeFAE) (Nieter *et al.* 2014) showed especially high substrate specificity for methyl sinapate. A feruloyl esterase of *Schizophyllum commune* (ScFAE) hydrolysed methyl sinapate, too, but not very efficiently (Nieter *et al.* 2016a). The aim of this study was to test if enzymatic degradation of kaempferol 3-*O*-*(2"'*-*O*-sinapoyl-β-sophoroside) to reduce the undesirable bitter off-taste of rapeseed protein isolate was possible.

### Materials and Methods

### Heterologous enzyme production

For heterologous enzyme production, *E. coli* BL21(DE3)Star was chosen to produce the SwFAE of prokaryotic origin, *Komagataella phaffii* GS115 was used for the PeFae, and *Komagataella phaffii* SMD 1168 for the ScFae1. Cultivations were performed according to Schulz *et al.* (2014) (SwFAE) and Nieter *et al.* (2016b). SwFaeD was produced using the cold shock expression vector pCOLD I DNA using different chaperons (TaKaRaMobitec, Göttingen, Germany). For expression, a shaking culture was inoculated in LB-media and incubated at 37 °C 0.1 mM isopropyl-β-D-thiogalactopyranosid (IPTG) was added to the culture, and the cells were cultivated at 17 °C and 200 rpm for 20 h, harvested, resuspended in 50 mM BisTris pH 7.0 and homogenized (precellys24 bead homogenizer). Pre-cultures of the *Komagataella* strains were grown at 28 °C on YEPD medium (10 g L-1 yeast extract, 20 g L-1 peptone, 20 g L-1 D-glucose). After three days, the medium was changed and main-cultures were grown on BMMY medium (10 g L-1 yeast extract, 20 g L-1 peptone, 100 mL L-1 potassium phosphate buffer (1 M, pH 6), 13.4 g L-1 yeast nitrogen base, 2 mL L-1 biotin, 50 mg L-1 each of L-glutamic acid, L-lysine, L-leucine, and L-isoleucine) at 20 °C. The expression was induced with 1% methanol (*v*/*v*). Every 24 h 2% methanol (*v*/*v*) was added to the cultures to ensure a continuous expression. After five days, *Komagataella* cultures were harvested by centrifugation (15,000 x g, 15 min, 4 °C) to separate the cell pellet. Afterwards, the supernatants were concentrated using ultrafiltration (Vivaspin^{®}, 10 kDa molecular mass cut off, Sartorius, Göttingen, Germany).

### Purification of SwFae, PeFae and ScFae1

The purification of the recombinant SwFae was conducted via Ni-NTA-agarose affinity chromatography. The lysate was incubated for 1 h in binding buffer (0.5M NaCl, 20mM Tris pH 7.9, and 5mM imidazole) at 4 °C while shaking. The sample was washed four times with 4 increasing imidazole concentrations, eluted with 500mM imidazole, and desalted using Sephadex G-25 Medium (GE Healthcare, Freiburg, Germany).

As it was challenging to purify PeFae without losing much of its activity, the enzyme was used directly from the ultra-filtered supernatant (10 kDa cut-off, PES, Sartorius, Göttingen, Germany). The recombinant feruloyl esterase of *Schizophyllum commune* was purified from *Komagataella phaffii* culture supernatant via Ni-NTA affinity chromatography. Bed volume of the self-casted column was 15 mL (column diameter 1.8 cm). Fractions of 8 mL were collected and assayed for enzymatic activity over 60 min at 37 °C as described elsewhere (Nieter *et al.* 2016a). Binding buffer (A) consisted of 0.5 M NaCl, 5 mM imidazole and 20 mM Tris•HCl (pH 7.9). Protein was eluted stepwise with elution buffer (B: 0.5 M NaCl, 500 mM imidazole and 20 mM Tris•HCl pH 7.9) with following gradient: 56 mL 0% B, 40 mL 1% B, 24 mL 5% B, 16 mL 100% B and re-equilibrated within 24 mL (0% B). The flow rate was 1 mL min-1 (isocratic flow). Active fractions were pooled, concentrated to approximately 2 mL and diluted 1/1 (*v*/*v*) in Tris buffer (50 mM, pH 7.5)

### Extraction of kaempferol 3-O-(2"'-O-sinapoyl-β-sophoroside)

Following the protocol described by Hald *et al.* 2019, 300 g rapeseed protein isolate powder (Isolexx, Teuteburger Ölmühle GmbH, Ibbenbüren, Germany) was extracted three times with 500 mL methanol/water (50/50, *v*/*v*) by stirring for 30 min at ambient temperature. Afterwards, the combined extracts were centrifuged (5 min, 5000 rpm, Hettich, Rotina 460 R, Tuttlingen, Germany) and filtered. The solvent was removed under vacuum at 40 °C. After lyophilisation, an aliquot (1 g) of the residue was taken up in 50 mL water and applied on a C18 cartridge (Chromabond, Macherey-Nagel, Düren, Germany). The column was preconditioned with 70 mL methanol and water each. Stepwise elution with 75 mL water (fraction A), 75 mL methanol/water (30/70, *v*/*v*) (fraction B) and 75 mL methanol/water (50/50, *v*/*v*) (fraction C) was performed. Fractions A and B were discarded. Solvent of fraction C was removed under 5 reduced pressure using a rotary evaporator at 40 °C. The aqueous residue was lyophilized and stored at -20 °C.

### Enzymatic hydrolysis

5 mg mL-1 of the lyophilized fraction C was dissolved in water and filtered (syringe filter Chromafil^{®} RC-45/25, Macherey-Nagel, Düren, Germany). 65 µL of this solution was incubated with 10 µL sodium acetate buffer (500 mM, pH 5.0) and 25 µL PeFae solution (107 U L -1) or 10 µL Tris buffer (500 mM, pH 7.5) and 25 µL ScFae solution (124 U mL -1) for 4 h at 37 °C. The reaction was terminated by doubling the reaction volume with acetonitrile. A control sample was prepared with water instead of rapeseed protein isolate extract (fraction C).

### LC-MS/MS

For analysis of rapeseed bitter compounds high performance liquid chromatography coupled to a triple quadrupole mass analyzer was used (Varian 212 LC pump, 320 TQ-MS mass spectrometer). The analyses were conducted in the negative electrospray ionisation mode with a scan range of *m*/*z* 100 to 900. Additionally, collision induced dissociation (CID) MS2 was performed for parent ions at *m*/*z* 815 (daughter ion scan range *m*/*z* 815 to 100) and *m*/*z* 223 (daughter ion scan range *m*/*z* 223 to 100) with the following parameters: capillary voltage - 50 V, collision energy 30 eV, spray chamber temperature 50 °C, drying gas temperature 300 °C, drying gas pressure 124 kPa, CID gas pressure 200 mPa (argon), needle voltage - 4500 V. For HPLC, water and acetonitrile (MS-grade), both containing 0.1 % formic acid, were used as the mobile phase. Gradient elution was used for separation: 100% solvent A (0.1% formic acid in water) and 0% solvent B (0.1% formic acid in acetonitrile) (held for 2 min), 100 to 70% solvent A in 1 min (held for 5 min), 70 to 0% solvent A in 3 min (held for 25 min), 0 to 100% solvent A in 2 min (held for 2 min). A six-port valve equipped with a 20 µL sample loop was used for manual injection and the flow rate was 0.4 mL min-1. The MS system was coupled to an UV/Vis detector (Varian ProStar 325). UV/Vis-spectra were recorded at *λ* = 220 nm and *λ* = 320 nm.

### Nano-LC-qTof-MS

Accurate masses of compounds were determined using the nano-liquid chromatography system EASY-nLC II (Bruker Daltronik, Bremen, Germany) equipped with a 20-mm pre-column (C18-A1 3PCS; ThermoFisher Scientific, Dreieich, Germany) and a capillary column (0.15 x 250 mm) packed with Grace MS C18 (3 µm particles, 300 Å pore; Grace Discovery Sciences (Columbia, USA), eluted by a linear gradient (300 nL min-1) of water and acetonitrile [each with 0.1 % formic acid (v/v)] from 95 % water to 95 % acetonitrile within 25 min and hold for 15 min. The nano-LC system was connected to a maXis impact QTOF mass spectrometer (Bruker Daltronik, Bremen, Germany) equipped with a captive nanospray ion source for electrospray ionization in the positive mode. The orthogonal time-of-flight mass analyser was calibrated prior to analysis (Na-formiate cluster) and operated with a mass resolution > 30,000.

### Results

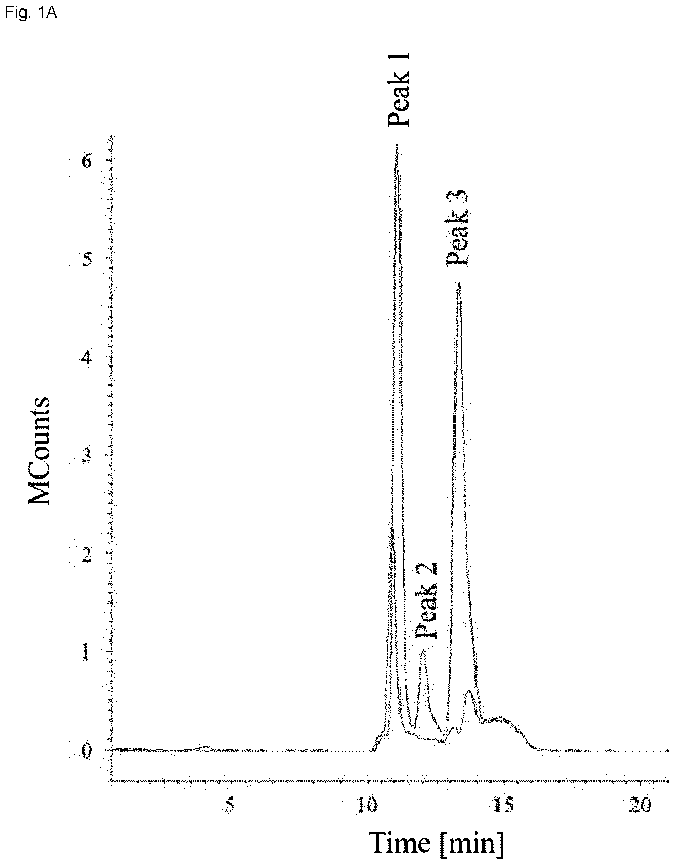

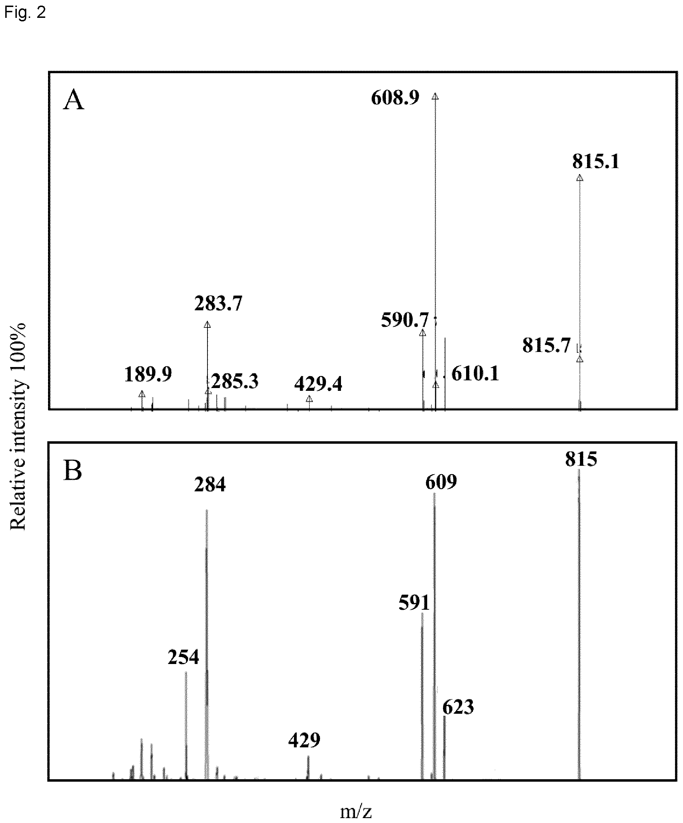

The SIM chromatogram of *m*/*z* 815 [M-H] showed three peaks in the untreated rapeseed protein extract (Fig. 1A). The accurate mass measurement of the peaks gave 815.2340, 815.2041, and 815.2052 (1 to 3), respectively. These masses correlate to an empirical formulae of C38H39020 (815.2029 calc.). By comparison of the mass spectra of the LC-MS/MS analysis with published reference spectra, peak 1 was identified as the key bitter compound kaempferol-3-*O*-(2"'-*O*-sinapoyl-*β*-sophoroside) (Fig. 2; Hald et al. 2019). A concluding identification of peak 2 was not possible, but based on accurate mass and MS/MS spectrum (no daughter ion at *m*/*z* 653) it is suggested that this was most likely a sophoroside derivative of kaempferol. Peak 3 was identified as kaempferol-3-O-sinapylglucoside-7-O-glucoside. The daughter ion at *m*/*z* 653 resulted from the cleavage of the sugar moiety in position 7 of kaempferol, indicating that this compound was not a sophoroside. This compound was found in rapeseed previously (Shao et al. 2014).

Enzymatic hydrolysis of kaempferol 3-*O*-*(2"'*-*O*-sinapoyl-*β*-sophoroside) using SwFae or PeFae showed little success. Looking at the measured activity against methyl sinapate these results were unexpected (Table 1).

**Table 1 Hydrolysis of Methyl sinapate and of kaempferol 3-O-(2"'-O-sinapoyl-β-sophoroside) by enzymes of different origins**

| Enzyme | highest activity against | relative activity against methyl sinapate | activity against kaempferol sophoroside |
|---|---|---|---|
| SwFae | Feruloyl arabinose | 47.13 ± 1.04 | n. d. |
| PeFae | β-D-Xylopyranosyl-(1→2)-5-O-trans-feruloyl-L-arabinofuranose | 4.6 ± 0.13 | n. d. |
| ScFae1 | Methyl p-coumarate | 3.0 ± 0.07 | high (Fig. 1) |

| | | | |
|---|---|---|---|
| n. d. = not detected | | | |

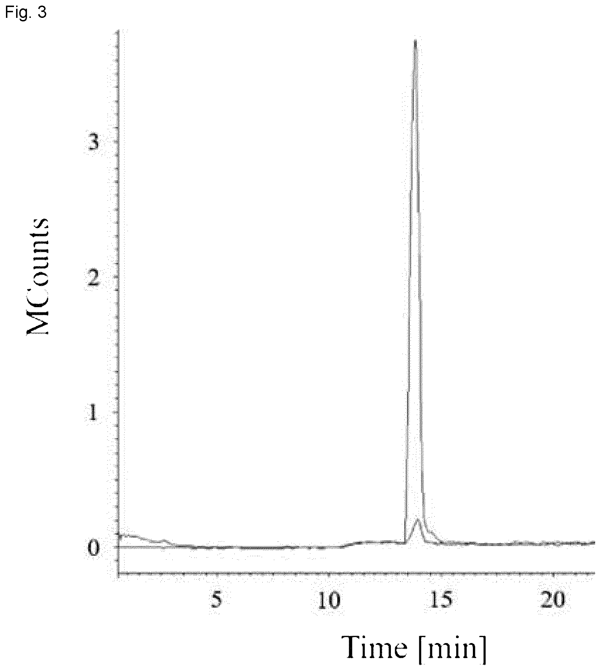

Different incubation temperatures and times did not appear to lead to improved result (data not shown). Treatment of the rapeseed protein extract with ScFae1 led to a decrease of 65 % of kaempferol 3-*O*-*(2"'*-*O*-sinapoyl-*β*-sophoroside) under the conditions applied (Fig. 1A, peak 1). Scanning *m*/*z* 223 showed that the sinapinic acid concentration increased simultaneously (Fig 3).

The two other substances were nearly completely hydrolyzed (peak 2: 99, % peak 3: 93 % of the peak area of the control sample, respectively). The hydrolysis of the ester bond of the kaempferol glycosides was additionally proven by the detection of two peaks showing accurate masses of 609.1468 and 609.1476, respectively, which corresponded to the kaempferol glycoside moieties of peak 1 and 3 (C27H29016, calc. 609.1455).

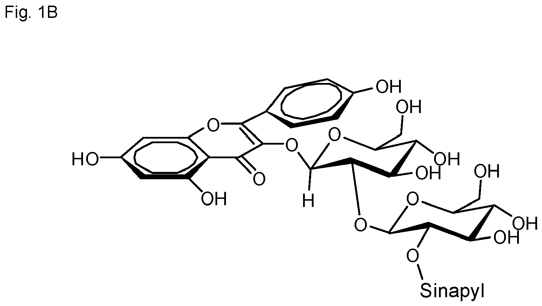

Surprisingly, even though neither SwFAE nor PeFAE, both with a very high relative activity toward methyl sinapate, showed hydrolytic activity towards kaempferol 3-O-(2"'-O-sinapoyl-β-sophoroside), ScoFAE cleaved the ester linkage between the sophoroside moiety and the sinapinic acid of kaempferol 3-*O*-*(2"'*-*O*-sinapoyl-β-sophoroside). This is an indication of the high substrate specificity required for this very bulky substrate (Fig. 1B).

### References

Calderón-Montaño J.M., Burgos-Morón E., Perez-Guerrero C. and Lopez-Lazaro M. (2011) A review on the dietary flavonoid kaempferol. Mini Reviews in Medicinal Chemistry. 11 205 (4): 298-344.
Crepin, V.F, Faulds, C.B. and Connerton, I.F. (2004) Functional classification of the microbial feruloyl esterases. Applied microbiology and Biotechnology 63(3), 647-52.
Hald, C., Dawid, C., Tressel, R. and Hofmann, T. (2019) Kaempferol 3-O-(2"'-O-Sinapoyl-β-sophoroside) Causes the Undesired Bitter Taste of Canola/Rapeseed Protein Isolates. Journal of Agricultural and Food Chemistry 67 (1), S. 372-378.
Kumar, S.P.J., Prasad, S.R., Banerjee, R., Agarwal, D.K., Kulkarni, K.S. and Ramesh, K. V. (2017) Green solvents and technologies for oil extraction from oilseeds. Chemistry Central Journal 11(9).
Nieter, A., Haase-Aschoff, P., Linke, D., Nimtz, M. and Berger, R.G. (2014) A halotolerant type A feruloyl esterase from Pleurotus eryngii. Fungal Biology 118(3), S. 348-357.
Nieter, A., Kelle, S., Linke, D. and Berger, R.G. (2016a) Feruloyl esterases from Schizophyllum commune to treat food industry side-streams. Bioresource Technology 220, 38-46.
Nieter, A., Kelle, S., Takenberg, M., Linke, D., Bunzel, M., Popper, L. and Berger, R.G. (2016b) Heterologous production and characterization of a chlorogenic acid esterase from Ustilago maydis with a potential use in baking. Food Chemistry 209, 1-9.
Schulz, K., Nieter, A., Scheu, A.-K., Copa-Patiño, J.L., Popper, L. and Berger, R.G. (2018) A type D ferulic acid esterase from Streptomyces werraensis affects the volume of wheat dough pastries. Appl. Microbiol. Biotechnol. 102(3), 1269-1279.
Shao, Y., Jiang, J., Ran, L., Lu, C., Wei, C. and Wang, Y. (2014) Analysis of Flavonoids and Hydroxycinnamic Acid Derivatives in Rapeseeds (Brassica napus L. var. napus) by HPLC-PDA-ESI(-)-MSn/HRMS. Journal of Agricultural and Food Chemistry 62(13), S. 2935-2945.
Siebert, M., Detering T. and Berger, R.G. (2018) An immobilized fungal chlorogenase rapidly degrades chlorogenic acid in a coffee beverage without altering its sensory properties. LWT--Food Science and Technology 2019, 115, 108426.
US Departments of Agriculture and USDA Foreign Agricultural Service (2019) Consumption of vegetable oils worldwide from 2013/14 to 2017/2018, by oil type (in million metric tons). https://www.statista.com/statistics/263937/vegetable-oils-global-consumption/10.01.2020
WO 2016 026793 A1

## Claims

1. A method of preparing a feed, comprising contacting an agro-industrial by-product with an enzyme capable of hydrolyzing the ester bond in Kaempferol 3-*O*-*(2"'*-*O-*sinapoyl-β-sophoroside) and comprising an amino acid sequence having at least 80% amino acid sequence identity to SEQ ID NO: 1, wherein an ester of kaempferol-3-*O*-*(2"'*-*O*-sinapoyl-β-sophoroside) is hydrolyzed.

2. A method of hydrolyzing an ester of kaempferol-3-*O*-*(2"'*-*O*-sinapoyl-β-sophoroside), the method comprising contacting kaempferol-3-*O*-*(2"'*-*O*-sinapoyl-β-sophoroside) with an enzyme capable of hydrolyzing the ester bond in kaempferol 3-*O*-*(2"'*-*O-*sinapoyl-β-sophoroside) and comprising an amino acid sequence having at least 80% amino acid sequence identity to SEQ ID NO: 1, wherein the method optionally is a method of claim 1.

3. A method of preparing a product selected from the group comprising
a. sinapinic acid; and/or
b. a kaempferol sophoroside moiety;
the method comprising contacting the educts of the catalyzed reaction generating the respective product under suitable conditions with an enzyme capable of hydrolyzing the ester bond in kaempferol 3-*O*-*(2"'*-*O*-sinapoyl-β-sophoroside) and comprising an amino acid sequence having at least 80% amino acid sequence identity to SEQ ID NO: 1, wherein the enzyme preferably contacts a natural material comprising said educts, such as an agro-industrial by-product.

4. The method of any of claims 1, 2 or 3, wherein the enzyme further has feruloyl esterase activity and/or chlorogenic acid esterase activity.

5. The method of claim 4, wherein the enzyme is obtainable from a basidiomycete, optionally, from *S*. *commune.*

6. The method of any of the preceding claims, wherein the enzyme is selected from the group consisting of enzymes comprising a sequence of SEQ ID NO: 1 and SEQ ID NO: 4.

7. The method of any of the preceding claims, wherein the enzyme comprises a sequence of SEQ ID NO: 1.

8. The method of any of the preceding claims, wherein the agro-industrial by-product comprises kaempferol-3-*O*-*(2"'*-*O*-sinapoyl-*β*-sophoroside).

9. The method of any of the preceding claims, wherein the agro-industrial by-product is obtainable during production of a vegetable oil, wherein the agro-industrial by-product preferably is a seed meal press cake.

10. The method of claim 9, wherein the agro-industrial by-product is a rapeseed meal press cake, optionally, a canola meal press cake.

11. The method of any of the preceding claims, wherein the bitterness of the feed is reduced.

12. The method of any of the preceding claims, wherein feed is food suitable for human consumption.

13. Use of an enzyme for reducing the bitterness of an agro-industrial by-product, wherein the enzyme is capable of hydrolyzing kaempferol 3-*O*-*(2"'*-*O*-sinapoyl-β-sophoroside) and comprises an amino acid sequence having at least 80% amino acid sequence identity to SEQ ID NO: 1

14. Use of claim 13, wherein the method of any of claims 1-2 or 4-12 is carried out.

15. A feed obtainable by the method of any of claims 1-2 or 4-12, and comprising an enzyme comprising an amino acid sequence having at least 80% amino acid sequence identity to SEQ ID NO: 1 that is optionally capable of hydrolyzing kaempferol 3-*O*-*(2"'*-*O*-sinapoyl-β-sophoroside).
